# FASCICULE DE BREVET EUROPEEN

(11) **EP 1 299 103 B1**
(45) Date de publication et mention de la délivrance du brevet: **28.09.2005**
(21) Numéro de dépôt: 01943579.1
(22) Date de dépôt: 07.06.2001
(51) Int. Cl.: A61K 31/426, A61P 25/00, C07D 277/18

(54) **DERIVES DE 4,5-DIHYDRO-THIAZOL-2-YLAMINE ET LEUR UTILISATION COMME INHIBITEURS DE NO SYNTHASE**
4,5-DIHYDRO-2-THIAZOLYLAMINDERIVATE UND IHRE VERWENDUNG ALS NO-SYNTHASE INHIBITOREN
4,5-DIHYDRO-THIAZO-2-YLAMINE DERIVATIVES AND THEIR USE AS NO-SYNTHASE INHIBITORS

(30) Priorité: 09.06.2000 FR 0007396
(43) Date de publication de la demande: 09.04.2003
(73) Titulaire: Aventis Pharma S.A., 92160 Antony (FR)
(72) Inventeur: CARRY, Jean-Christophe, F-94100 Saint Maur des Fosses (FR); DAMOUR, Dominique, F-94310 Orly (FR); GUYON, Claude, F-94100 Saint Maur des Fosses (FR); MIGNANI, Serge, F-92290 Chatenay-Malabry (FR); BACQUE, Eric, F-91190 Gif sur Yvette (FR); BIGOT, Antony, F-91300 Massy (FR)
(74) Mandataire: Rousseau, Pierrick Edouard
(86) Numéro de dépôt international: PCT/FR2001/001759
(87) Numéro de publication internationale: WO 2001/093867

(56) Documents cités:
- WO-A-94/12165
- WO-A-95/11231
- WO-A-96/14842
- CHEMICAL ABSTRACTS SERVICE, REGISTRY HANDBOOK, NUMBER SECTION, 1981, page 720RJ,

## Description

La présente invention concerne les compositions pharmaceutiques contenant en tant que principe actif un dérivé de 4,5-dihydro-1,3-thiazol-2-ylamine de formule : 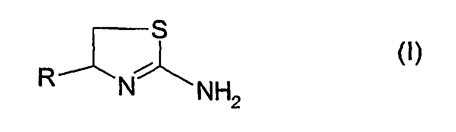 ou un de ses sels pharmaceutiquement acceptables, les nouveaux dérivés de formule (I) et leur préparation.

Les composés de formule (I) sont des inhibiteurs de la synthase du monoxyde d'azote et particulièrement de l'isoforme inductible de cette enzyme.

Le monoxyde d'azote (NO) est un radical diffusible impliqué dans de nombreux processus physiologiques et pathologiques. Il est synthétisé par oxydation de la L-arginine, une réaction catalysée par une famille d'enzymes appelée synthase du monoxyde d'azote ou NO-Synthase (NOS), référencée dans le système international de nomenclature des enzymes sous le numéro E.C. 1.14.13.39.

Trois isoformes de NOS, dont deux sont constitutives et une inductible, sont connues :
- une NOS neuronale (NOS-1 ou nNOS) a été isolée et clonée à l'origine à partir de tissu nerveux où c'est une enzyme constitutive. La NOS-1 produit du NO en réponse à divers stimuli physiologiques tels que l'activation de récepteurs membranaires selon un mécanisme dépendant du calcium et de la calmoduline.
- une NOS inductible (NOS-2 ou iNOS) peut être induite en réponse à des stimuli immunologiques tels que par exemple les cytokines ou des antigènes bactériens dans différentes cellules telles que par exemple les macrophages, les cellules endothéliales, les hépatocytes, les cellules gliales, ainsi qu'un grand nombre d'autres types de cellules, L'activité de cette isoforme n'est pas régulée par le calcium. C'est pourquoi une fois induite elle produit de grandes quantités de NO sur des durées prolongées.
- une NOS endothéliale (NOS-3 ou eNOS) est constitutive et calcium/calmoduline dépendante. Elle a été identifiée à l'origine dans les cellules de l'endothélium vasculaire où elle génère du NO en réponse à des stimuli physiologiques tels que l'activation de récepteurs membranaires.

Le NO produit par les isoformes constitutives neuronales et endothéliales (NOS-1 et NOS-3) est généralement impliqué dans des fonctions de signalisation intercellulaire. Par exemple, les cellules endothéliales qui tapissent la paroi interne des vaisseaux sanguins induisent la relaxation des cellules musculaires lisses sous-jacentes *via* la production de NO. Il contribue ainsi à la régulation de la pression artérielle.

Le NO produit en grande quantité par l'isoforme inductible NOS-2 est, entre autre, impliqué dans les phénomènes pathologiques associés aux processus inflammatoires aigus et chroniques dans une grande variété de tissus et d'organes.

Une production excessive de NO par induction de NOS-2 participe ainsi de pathologies dégénératives du système nerveux comme par exemple la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie. De même, en dehors du système nerveux central, l'induction de la NOS-2 est impliquée dans de nombreuses pathologies à composantes inflammatoires comme par exemple le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis. La NOS-2 a également été impliquée dans la croissance de certaines formes de tumeurs comme par exemple des épithéliomes, des adénocarcinomes ou des sarcomes, et dans les infections par des bactéries intracellulaires ou extracellulaires, Gram-plus ou Gram-moins.

Dans toutes les situations où une production anormale de NO est néfaste, il apparaît donc souhaitable de diminuer la production de NO par l'administration de substances capables d'inhiber la NOS-2.

Des inhibiteurs de NOS dérivés de thiazoline sont notamment décrits dans les demandes de brevet WO94/12165, WO95/11231 et WO96/14842.

Les compositions pharmaceutiques selon la présente invention sont celles contenant en tant que principe actif un dérivé de formule (I) dans laquelle R représente un radical -alk-S-alk-Ar, phényle ou phényle substitué par alcoxy ou halogène, Ar est un radical phényle et alk représente un radical alkylène.

Lorsque R est un phényle substitué celui-ci est, de préférence, monosubstitué et, en particulier, en position -3 ou -4.

Dans les définitions précédentes et celles qui suivent, les radicaux alkyle, alkylène, alcoxy et les portions alkyle et alcoxy contiennent 1 à 6 atomes de carbone en chaîne droite ou ramifiée.

Les atomes d'halogène sont les atomes de brome, chlore, iode et fluor et, plus particulièrement l'atome de brome.

Les radicaux alcoxy sont notamment les radicaux méthoxy, éthoxy, propoxy et, plus préférentiellement méthoxy.

De préférence, R représente un radical phényle, phényle monosubstitué par alcoxy et plus particulièrement méthoxy ou par un atome d'halogène et plus particulièrement de brome.

Les composés de formule (I) présentent un ou plusieurs atomes de carbone asymétriques et peuvent donc se présenter sous forme de racémiques, d'énantiomères et de diastéréoisomères; ceux-ci font également partie de l'invention ainsi que leurs mélanges.

Par ailleurs les composés de formule (I) peuvent se présenter sous la forme tautomère (Ia) :

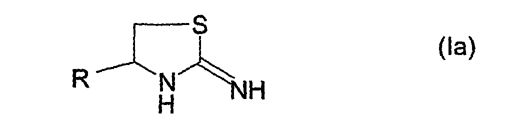

Ces tautomères font également partie de l'invention.

Les compositions pharmaceutiques préférées, sont celles contenant un composé de formule (I), ses racémique, énantiomères et diastéréoisomères, son tautomère ainsi que ses sels pharmaceutiquement acceptables, choisi parmi les composés suivants :
4-(3-bromophényl)-4,5-dihydro-1,3-thiazol-2-ylamine
4-(4-méthoxyphényl)-4,5-dihydro-1,3-thiazol-2-ylamine
4-phényl-4,5-dihydro-1,3-thiazol-2-ylamine
4-(benzylsulfanylméthyl)-4,5-dihydro-1,3-thiazol-2-ylamine.

Sont encore plus préférées les compositions pharmaceutiques contenant en tant que principe actif un composé de formule (I), son tautomère ou ses sels pharmaceutiquement acceptables, choisi parmi les composés suivants :
(4RS)-4-(3-bromophényl)-4,5-dihydro-1,3-thiazol-2-ylamine
4-(4-méthoxyphényl)-4,5-dihydro-1,3-thiazol-2-ylamine
(-)-(4R) -4-(4-méthoxyphényl)-4,5-dihydro-1,3-thiazol-2-ylamine
(-)-(4R)-4-phényl-4,5-dihydro-1,3-thiazol-2-ylamine.

Le dérivé de formule (I) pour lequel R est phényle est connu (Chem. Abst., registry Number 76999-87-6).

Les autres dérivés de formule (I) sont nouveaux et font partie de l'invention en tant que tels.

Les composés de formule (I) préférés sont les composés suivants :
4-(3-bromophényl)-4,5-dihydro-1,3-thiazol-2-ylamine
4-(4-méthoxyphényl)-4,5-dihydro-1,3-thiazol-2-ylamine
4-(benzylsulfanylméthyl)-4,5-dihydro-1,3-thiazol-2-ylamine
leurs racémiques, énantiomères, diastéréoisomères et leurs mélanges, leurs tautomères et leurs sels pharmaceutiquement acceptables.

Encore plus préférés sont les composés suivants :
(4RS)-4-(3-bromophényl)-4,5-dihydro-1,3-thiazol-2-ylamine
4-(4-méthoxyphényl)-4,5-dihydro-1,3-thiazol-2-ylamine
(-)-(4R) -4-(4-méthoxyphényl)-4,5-dihydro-1,3-thiazol-2-ylamine
leurs tautomères et leurs sels pharmaceutiquement acceptables.

Les composés de formule (I) peuvent être préparés par cyclisation d'un dérivé de formule : 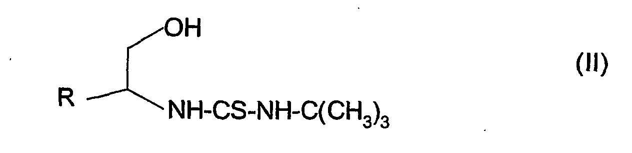 dans laquelle R a les mêmes significations que dans la formule (I).

Cette cyclisation s'effectue généralement au moyen d'un acide tel que l'acide chlorhydrique, en milieu aqueux, à une température de 100°C. De préférence, on utilise l'acide chlorhydrique 6N.

Les dérivés de formule (II) peuvent être obtenus selon le schéma réactionnel suivant :

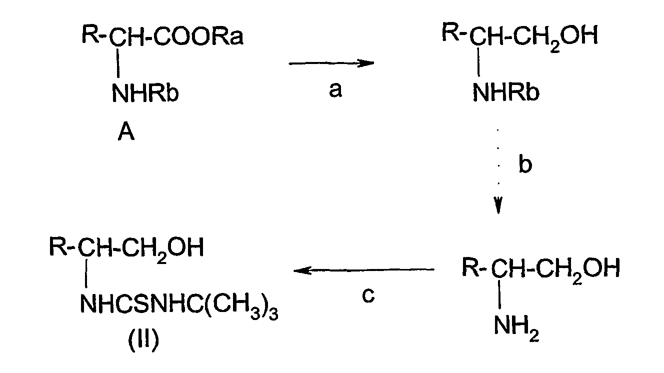

Dans ces formules R a les mêmes significations que dans la formule (I), Ra représente un atome d'hydrogène ou un radical alkyle ou alcoxycarbonyle, de préférence, méthyle, éthyle ou isobutyloxycarbonyle et Rb est un atome d'hydrogène ou un groupe protecteur de la fonction amine tel que ceux décrits par T.W. GREENE, Protective groups in Organic Synthesis, J. Wiley-Interscience Publication (1991) et, de préférence un radical acétyle ou tert-butyloxycarbonyle.

La réduction de l'étape a s'effectue de préférence au moyen d'un hydrure tel que le borohydrure de sodium ou l'aluminohydrure de lithium, au sein d'un alcool aliphatique (1-4C) ou du tétrahydrofuranne, à une température comprise entre 10°C et 30°C, ou bien au moyen d'un dérivé du borane tel que le complexe BH₃-THF, au sein d'un solvant tel que le tétrahydrofuranne, à une température comprise entre 0°C et 30°C.

La réaction de déprotection b pour les composés pour lesquels Rb est un groupe protecteur de la fonction amine s'effectue par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température de 100°C. Lorsque le groupe protecteur est un radical tert-butyloxycarbonyle cette réaction s'effectue au moyen d'acide chlorhydrique au sein du dioxanne, à une température voisine de 20°C.

La réaction c s'effectue par action du tert-butyl isothiocyanate, au sein d'un solvant inerte tel qu'un alcool aliphatique (1-4C) (méthanol, éthanol de préférence), en présence d'une amine tertiaire telle que la triéthylamine, à une température comprise entre 20°C et la température d'ébullition du milieu réactionnel.

Les intermédiaires A sont commercialisés ou peuvent être préparés par application ou adaptation des méthodes décrites dans les exemples et notamment par les méthodes suivantes :

Lorsque R est phényle substitué par halogène, l'intermédiaire A peut être obtenu à partir de l'halogénobenzaldéhyde correspondant par action d'hydroxyde de potassium et d'ammoniaque, en présence de chlorure de lithium et de chlorure de benzyltriéthylammonium, au sein d'un solvant tel qu'un mélange de dichlorométhane, de chloroforme et d'eau à une température comprise entre 0°C et 30°C, suivie éventuellement d'une estérification par action d'un alcool aliphatique (1-4C) (méthanol, éthanol de préférence), en présence d'un acide minéral tel que l'acide sulfurique, à une température comprise entre 50°C et la température d'ébullition du milieu réactionnel. Lorque R est phényle substitué par alcoxy, Ra est un radical alkyle et Rb est tert-butyloxycarbonyle, l'intermédiaire A peut être obtenu par alkylation de la N-tert-butyloxycarbonyl-hydroxyphénylglycine correspondante par action d'un halogénure d'alkyle (iodure de méthyle par exemple), en présence d'une base telle que le carbonate de potassium, au sein d'un solvant inerte tel que le diméthylformamide, à une température comprise entre 0°C et 30°C.

Les composés de formule (I) pour lesquels R est un radical -alk(1C)-S-alk-Ar peuvent également être préparés par action d'un dérivé de formule : 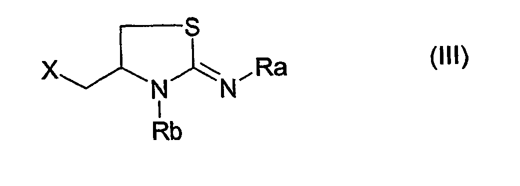 dans laquelle X est un atome d'halogène et, de préférence, iode ou un radical tosyle, Ra et Rb sont des atomes d'hydrogène ou des groupes protecteurs de la fonction amine tels que ceux décrits par T.W. GREENE, Protective groups in Organic Synthesis, J. Wiley-Interscience Publication (1991), de préférence alcoxycarbonyle ou acétyle et plus particulièrement tert-butyloxycarbonyle, avec un dérivé de formule HS-alk-Ar dans laquelle Ar représente un radical phényle et alk est un radical alkylène (1-6C en chaîne droite ou ramifiée, suivie si nécessaire d'une déprotection de la fonction amine.

Cette réaction s'effectue généralement en présence d'une base telle que le carbonate de potassium, au sein d'un solvant tel que l'acétonitrile ou le diméthylformamide et de préférence l'acétonitrile, à une température comprise entre 20°C et la température d' ébullition du milieu réactionnel.

La réaction de déprotection pour les composés pour lesquels Ra ou Rb est un groupe protecteur de la fonction amine s'effectue par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température de 100°C. Lorsque le groupe protecteur est un radical tert-butyloxycarbonyle cette réaction s'effectue au moyen d'acide chlorhydrique au sein du dioxanne, à une température voisine de 20°C.

Les composés de formule (III) peuvent eux-mêmes être obtenus selon le schéma réactionnel suivant :

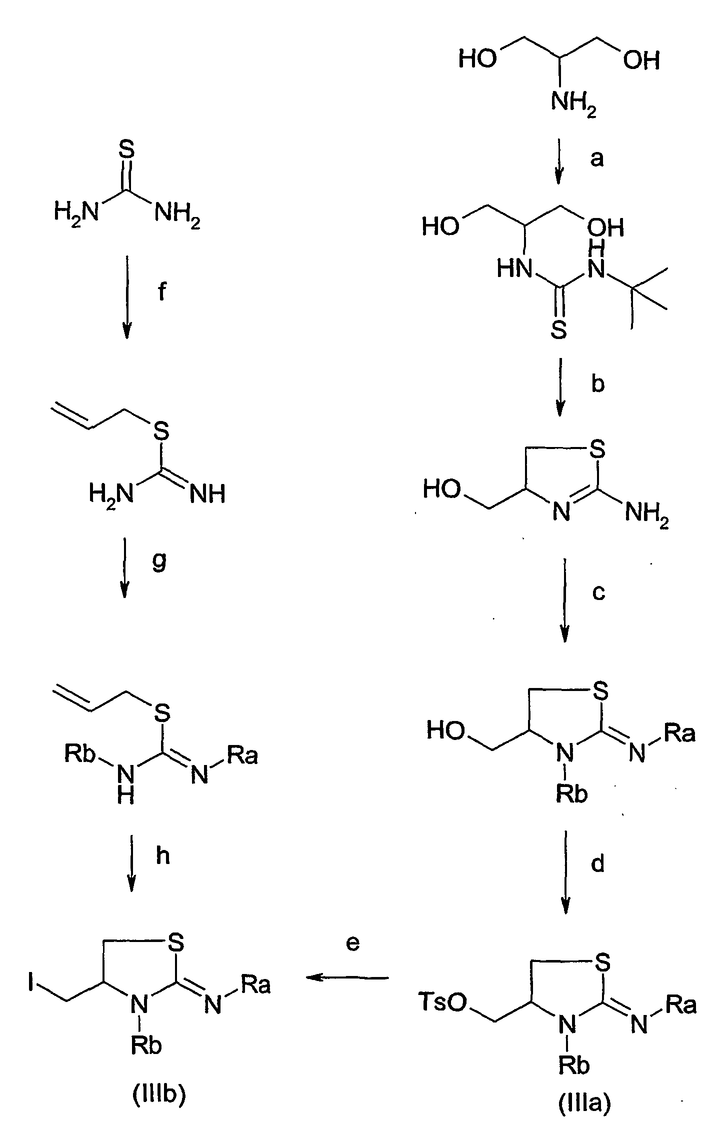

Dans ces formules, Ra et Rb sont un atome d'hydrogène ou un groupe protecteur de la fonction amine tel que ceux décrits par T.W. GREENE, Protective groups in Organic Synthesis, J. Wiley-Interscience Publication (1991), de préférence alcoxycarbonyle ou acétyle et plus particulièrement tert-butyloxycarbonyle et Ts est un radical tosyle.

La réaction a s'effectue généralement par action du tert-butyl isothiocyanate, au sein d'un solvant inerte tel qu'un alcool aliphatique (1-4C) (méthanol, éthanol de préférence), éventuellement en présence d'une amine tertiaire telle que la triéthylamine, à une température comprise entre 20°C et la température d'ébullition du milieu réactionnel.

La réaction de cyclisation b s'effectue généralement au moyen d'un acide tel que l'acide chlorhydrique, en milieu aqueux, à une température voisine de 100°C. De préférence, on utilise l'acide chlorhydrique 6N.

Les réactions c et g lorsque Ra ou Rb est un groupe tert-butyloxycarbonyle s'effectuent par toute méthode de protection connue de l'homme de l'art et notamment celles décrites par T.W. GREENE, Protective groups in Organic Synthesis, J. Wiley-Interscience Publication (1991). De préférence cette réaction s'effectue au moyen de dicarbonate de bis-tert-butyle, en présence d'une base telle que la triéthylamine et éventuellement en présence de 4-(diméthylamino)pyridine, au sein d'un solvant tel que le dichlorométhane et à une température voisine de 20°C, ou bien en présence d'une base telle que le carbonate de potassium, au sein d'un solvant tel que l'eau et à une température voisine de 20°C.

La réaction d s'effectue généralement par action du chlorure de p-toluène sulfonyle, en présence d'une amine tertiaire telle que la triéthylamine, au sein d'un solvant inerte tel que le dichlorométhane, à une température comprise entre 20°C et la température d'ébullition du milieu réactionnel.

La réaction e s'effectue généralement par action de l'iodure de sodium, au sein d'un solvant inerte tel que l'acétone, à une température comprise entre 20°C et la température d'ébullition du milieu réactionnel.

La réaction f s'effectue généralement par action d'un halogénure d'allyle, par exemple le chlorure d'allyle, au sein d'un alcool aliphatique (1-4C), de préférence l'éthanol, à une température comprise entre 20°C et la température d'ébullition du milieu réactionnel.

La réaction h s'effectue généralement par action d'iode, en présence d'une base telle que le bicarbonate de sodium, au sein d'un solvant tel que le dichlorométhane, à une température comprise entre 20°C et la température d'ébullition du milieu réactionnel.

Les composés de formule (I) sont isolés et peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) sont des inhibiteurs de NO-synthase inductible ou NO-synthase de type 2 (NOS-2) et sont ainsi utiles pour la prévention et le traitement des désordres liés à une production excessive de NO telles que la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis, la croissance de certaines formes de tumeurs comme par exemple les épithéliomes, les adénocarcinomes ou les sarcomes, et les infections par des bactéries intracellulaires ou extracellulaires, gram-plus ou gram-moins.

Leur activité en tant qu'inhibiteurs de la NOS-2 a été déterminée par la mesure de la conversion de [³H]-L-arginine en [³H]-L-citrulline par une fraction enzymatique NOS-2 extraite de poumons de rats ou de souris préalablement traités par des lipopolysaccharides (10 mg/kg i.p. 6 heures avant le recueil de tissu. Les composés ont été incubés pendant 20 à 30 minutes à 37°C en présence de 5 µM de [³H]-L-arginine, 1 mM de NADPH, 15 µM de tétrabiopterine, 1µM de FAD, 0.1 mM de DTT dans un tampon HEPES (50 mM, pH 6,7) contenant 10 µg/ml de calmoduline. L'incubation a été arrêtée par addition de tampon HEPES froid (100 mM, pH 5,5) contenant 10 mM d'EGTA et 500 mg d'une résine cationique échangeuse d'ion (AG50W-X8, contre-ion: Na⁺) pour séparer la [³H]-L-arginine de la [³H]-L-citrulline. Après 5 minutes de décantation, la radioactivité restant dans la phase liquide a été mesurée dans un compteur à scintillation en présence d'un liquide scintillant approprié. Le rendement de la récupération de la L-[³H]citrulline formée a pu être estimée en utilisant de la L-[ureido-¹⁴C]-citrulline comme standard externe.

L'activité a été exprimée en picomole(s) de [³H]-L-citrulline formée par minute et par milligramme de protéine contenu dans le milieu réactionel.

Dans ce test, la CI₅₀ des composés de formule (I) est inférieure ou égale à 1 µM.

Les composés de formule (I) présentent une toxicité faible. Leur DL₅₀ est supérieure à 40 mg/kg par voie sous cutanée chez la souris.

Les exemples suivants illustrent l'invention.

### Exemple 1

### Chlorhydrate de (4RS)-4-(3-bromophényl)-4,5-dihydro-1,3-thiazol-2-ylamine

Une suspension de 0,5 g de N-(*tert*-butyl)-N'-[2-hydroxy-1-(3-bromophényl)éthyl]thiourée dans 3,8 cm³ d'acide chlorhydrique 6N est chauffée sous agitation à une température voisine de 100°C pendant 5 heures 30 minutes. Le milieu réactionnel est alors refroidi à une température voisine de 20°C. Il se forme un précipité blanc que l'on filtre après 30 minutes d'agitation. Le gâteau est rincé par de l'éther diéthylique, puis séché à l'étuve sous pression réduite (10 Pa) à une température voisine de 60°C, puis est purifié par chromatographie, sous une pression d'argon de 50 kPa, sur une colonne de gel de silice (granulométrie 40-63µ ; diamètre 1,5 cm ; hauteur 20 cm) en éluant par un mélange de dichlorométhane-méthanol (90/10 en volumes). On recueille les fractions contenant le produit attendu. Celles-ci sont réunies puis concentrées sous pression réduite (5 kPa) à une température voisine de 40°C. On obtient 0,1 g de chlorhydrate de (4RS)-4-(3-bromophényl)-4,5-dihydro-1,3-thiazol-2-ylamine, sous forme d'un solide blanc (R^{f}=0,26 dans le mélange dichlorométhane-méthanol 90/10 en volumes, sur plaque de silice Merck 60F_{254^{R}}); [Spectre de R.M.N. ¹H (250 MHz, (CD₃)₂SO d6, δ en ppm) : 3,45 (dd, J = 14 et 8 Hz : 1H) ; 3,97 (dd, J = 14 et 8,5 Hz : 1H) ; 5,42 (dd, J = 8,5 et 8 Hz : 1H) ; de 7,35 à 7,55 (mt : 2H) ; de 7,55 à 7,75 (mt : 2H) ; de 9,10 à 10,90 (mf étalé : 1H)].

N-(*tert*-Butyl)-N'-[2-hydroxy-1-(3-bromophényl)éthyl]thiourée : Une solution de 3,54 g de 2-amino-2-(3-bromophényl)-1-éthanol, dans 20 cm³ d'éthanol additionnée de 0,18 cm³ d'isothiocyanate de *tert*-butyle est agitée à une température voisine de 20°C pendant 5 heures. Après concentration de la masse réactionnelle sous pression réduite (5 kPa) à une température voisine de 40°C, le résidu obtenu est repris par 25 cm³ d'éther de pétrole. Les cristaux qui en résultent sont essorés, lavés par 2 fois 25 cm³ d'éther de pétrole puis séchés sous pression réduite (5 kPa) à une température voisine de 20°C. On obtient 4 g de N-(*tert*-butyl)-N'-[2-hydroxy-1-(3-bromophényl)éthyl]thiourée, sous forme d'un solide blanc (R^{f}=0,68 dans le mélange dichlorométhane-méthanol-ammoniaque 40/5/0,5 en volumes, sur plaque de silice Merck 60F_{254^{R}}).

2-Amino-2-(3-bromophényl)-1-éthanol : Une solution de 4,4 g de 3-bromophénylglycinate d'éthyle dans 80 cm³ d'éthanol est maintenue à une température voisine de 20°C. On ajoute sous agitation, par petites fractions, 0,97 g de borohydrure de sodium, puis le mélange est agité pendant 18 heures à une température voisine de 20°C. Après concentration du milieu réactionnel sous pression réduite (5 kPa) à une température voisine de 40°C, le résidu obtenu est repris par 40 cm³ d'eau, puis extrait par 3 fois 50 cm³ d'acétate d'éthyle. Les extraits sont réunis puis séchés sur sulfate de sodium, filtrés, concentrés sous pression réduite (5 kPa) à une température voisine de 40°C. On obtient une huile que l'on purifie par chromatographie sous une pression d'argon de 50 kPa, sur une colonne de gel de silice (granulométrie 40-63µ ; diamètre 2,5 cm ; hauteur silice 35 cm), en éluant par un mélange de dichlorométhane-méthanol (90/10 en volumes). On recueille les fractions contenant le produit. Celles-ci sont réunies puis concentrées sous pression réduite (5 kPa) à une température voisine de 40°C. On obtient 2,1 g de 2-amino-2-(3-bromophényl)-1-éthanol, sous forme d'un solide de couleur jaune fondant à 76°C.

3-Bromophénylglycinate d'éthyle : Un mélange de 21,3 g de 3-bomophénylglycine dans 160 cm³ d'éthanol chlorhydrique 6,5N est chauffé sous agitation pendant 24 heures à une température voisine de 80°C. Le milieu réactionnel est filtré, puis le gâteau lavé par 2 fois 30 cm³ d'éthanol. Le filtrat est concentré sous pression réduite (5 kPa) à une température voisine de 40°C. On obtient une huile que l'on alcalinise par addition d'une solution aqueuse de carbonate de sodium. Le mélange est extrait par 3 fois 100 cm³ d'acétate d'éthyle. Les extraits sont réunis, lavés par 2 fois 100 cm³ d'une solution aqueuse de chlorure de sodium, séchés sur sulfate de sodium, filtrés, concentrés sous pression réduite (5 kPa) à une température voisine de 40°C. On obtient 4,7 g de 3-bromophénylglycinate d'éthyle, sous forme d'une huile de couleur jaune (R^{f}=0,38 dans le mélange dichlorométhane-méthanol 90/5 en volumes, sur plaque de silice Merck 60F_{254^{R}}).

3-Bromophénylglycine : A un mélange agité sous atmosphère inerte de 16,8 g de potasse dans 56 cm³ d'ammoniaque à 32%, on introduit 8,4 g de chlorure de lithium, puis 2,78 g de chlorure de benzyltriéthylammonium préalablement solubilisés dans 50 cm³ de dichlorométhane. Le mélange obtenu est refroidi à une température voisine de 0°C puis on y ajoute 18,5 g de 3-bromobenzaldéhyde préalablement solubilisés dans 50 cm³ de dichlorométhane et 12,8 cm³ de chloroforme, tout en maintenant le mélange à une température voisine de 0°C. Le milieu réactionnel est agité pendant 6 heures à cette température, puis 18 heures à une température voisine de 20°C. L'insoluble est filtré, puis le filtrat est décanté. La phase aqueuse est séparée, lavée par 2 fois 50 cm³ de dichlorométhane, acidifiée par 10 cm³ d'acide chlorhydrique concentré pour obtenir un pH de 7. On termine l'acidification jusqu'à pH 6 par une addition supplémentaire d'acide chlorhydrique aqueux 1N. L'acide attendu précipite après grattage. Le mélange est agité pendant une heure à une température voisine de 5°C, puis filtré. Les cristaux obtenus sont lavés par 5 cm³ d'eau, séchés à l'étuve sous pression réduite (10 Pa) à une température voisine de 60°C. On obtient 0,41 g de 3-bromophénylglycine, sous forme d'un solide blanc [Spectre infra rouge (KBr) : 3080 ; 2980 ; 2670 ; 1635 ; 1580 ; 1400 ; 1355 ; 775 et 695 cm⁻¹].

### Exemple 2

### Oxalate de 4-(4-méthoxyphényl)-4,5-dihydro-1,3-thiazol-2-ylamine

Un mélange de 1,3 g de N-*(tert*-butyl)-N'-[1-(4-méthoxyphényl)-2-hydroxyéthyl]thiourée dans 12,3 cm³ d'acide chlorhydrique aqueux 6N est chauffé sous agitation à une température voisine de 100°C pendant 1 heure. L'huile dense qui a précipité est séparée par décantation de la phase aqueuse chlorhydrique surnageante. Après refroidissement aux environs de 20°C, cette dernière est extraite par 5 cm³ d'acétate d'éthyle, puis la phase aqueuse résultante est évaporée sous pression réduite (5 kPa) à une température voisine de 50°C. On obtient une huile que l'on débarrasse de son eau résiduelle par entraînement azéotropique avec d'abord 10 cm³ d'éthanol, puis 10 cm³ d'éther diéthylique, chaque reprise étant suivie d'une concentration dans les conditions de ci-dessus. A l'huile obtenue on ajoute 6 cm³ de soude 1N, puis on extrait le mélange par 30 cm³ d'éther diéthylique additionnés de 15 cm³ d'acétate d'éthyle. La phase organique est décantée, évaporée sous pression réduite (5 kPa) à une température voisine de 40°C. Au résidu obtenu on ajoute 0,1 g d'acide oxalique préalablement solubilisé dans 1 cm³ d'acétone. Le précipité blanc formé est séparé par filtration, lavé 1 fois à l'acétone, 2 fois au chloroforme, puis 1 fois à l'acétate d'éthyle, séché sous pression réduite (10 Pa) à une température voisine de 40°C. On obtient 0,063 g d'oxalate de 4-(4-méthoxyphényl)-4,5-dihydro-1,3-thiazol-2-ylamine, sous forme d'un solide de couleur blanche fondant à 180°C [Spectre de R.M.N. ¹H (300 MHz, (CD₃)₂SO d6, δ en ppm) : 3,40 (dd, J = 14 et 8,5 Hz : 1H) ; 3,80 (s : 3H) ; 3,93 (dd, J = 14 et 8,5 Hz :1H) ; 4,22 (mf : 2H) ;3,58 (t, J = 8,5 Hz : 1H) ; 7,00 (d, J = 8,5 Hz : 2H) ; 7,34 (d, J =8,5 Hz : 2H)].

N-*(tert*-Butyl)-N'-[1-(4-méthoxyphényl)-2-hydroxyéthyl]thiourée : On procède dans les conditions de l'exemple 1 à partir de 1,3 g de 2-amino-2-(4-méthoxyphényl)-1-éthanol et de 1,46 cm³ d'isothiocyanate de *tert*-butyle dans 12 cm³ d'éthanol absolu pendant 21 heures à une température voisine de 20°C. Après concentration de la masse réactionnelle sous pression réduite (5 kPa) à une température voisine de 40°C, le résidu obtenu est repris par 10 cm³ d'eau. II se forme un précipité cristallisé qui est filtré, lavé par 3 fois 5 cm³ d'éther diéthylique, séché sous pression réduite (10 Pa) à une température voisine de 40°C. On obtient 1,32 g de N-*(tert*-butyl)-N'-[1-(4-méthoxyphényl)-2-hydroxyéthyl]thiourée, sous forme d'un solide de couleur blanche fondant à 127°C.

2-Amino-2-(4-méthoxyphényl)-1-éthanol : Un mélange de 3,4 g de 1-(4-méthoxyphényl)-2-hydroxyéthylcarbamate de *tert*-butyle dans 32 cm³ de méthanol à 10% en masse d'acide chlorhydrique anhydre, est agité pendant 1, heure à une température voisine de 20°C. Le milieu réactionnel est concentré sous pression réduite (5 kPa) à une température voisine de 40°C. Le résidu est repris par 9 cm³ d'une solution aqueuse à 5% d'hydrogénocarbonate de sodium, puis le mélange est extrait par 3 fois 30 cm³ de dichlorométhane. La phase aqueuse est concentrée comme ci-dessus, puis le solide blanc obtenu est repris par 17 cm³ d'une solution aqueuse de soude 1N. Le précipité est extrait par 3 fois 30 cm³ de dichlorométhane. Les extraits organiques réunis sont évaporés sous pression réduite (5 kPa) à une température voisine de 40°C. Le solide blanc obtenu est séché sous pression réduite (10 Pa) à une température voisine de 40°C. On obtient 1,3 g de 2-amino-2-(4-méthoxyphényl)-1-éthanol, sous forme d'un solide blanc fondant à 96°C.

1-(4-Méthoxyphényl)-2-hydroxyéthylcarbamate de tert-butyle : A une solution de 7,8 g de N-Boc-(4-méthoxyphényl)glycinate de méthyle dans 35 cm³ de tétrahydrofuranne refroidie à une température voisine de 0°C, on ajoute 2,24 g de chlorure de lithium, puis par petites fractions 1,99 g de borohydrure de sodium, et enfm 74 cm³ d'éthanol. On laisse remonter la température au voisinage de 20°C, puis la réaction est terminée par agitation du mélange pendant 18 heures à cette même température. Le milieu est à nouveau refroidi aux environs de 5°C, et l'on y ajoute la quantité suffisante d'une solution aqueuse d'hydrogénosulfate de sodium 1M pour obtenir un pH voisin de 2. Le mélange est agité pendant 3 heures, puis est évaporé sous pression réduite (5 kPa) à une température voisine de 50°C. Au résidu obtenu on ajoute 60 cm³ de dichlorométhane et 30 cm³ d'une solution aqueuse d'hydronénosulfate de sodium 1M. Après agitation du mélange puis décantation de la phase organique, la phase aqueuse est extraite par 2 fois 30 cm³ de dichlorométhane. L'ensemble des extraits organiques réunis est séché sur sulfate de sodium, concentré comme ci-dessus. On obtient un solide blanc que l'on reprend dans 30 cm³ de cyclohexane et que l'on filtre. Les cristaux sont séchés sous pression réduite (10 Pa) à une température voisine de 60°C. On obtient 3,47 g de 1-(4-méthoxyphényl)-2-hydroxyéthylcarbamate de tert-butyle, sous forme d'un solide blanc fondant à 130°C.

N-Boc-(4-méthoxyphényl)glycinate de méthyle : A une solution agitée de 8,25 g de N-Boc-(4-hydroxyphényl)glycine dans 112 cm³ de diméthylformamide anhydre et refroidie à une température voisine de 0°C, on ajoute 10,45 g de carbonate de potassium, puis goutte à goutte 4,7 cm³ d'iodure de méthyle. Le mélange est agité à une température voisine de 20°C pendant 18 h 30. Après addition de 280 cm³ d'éther diéthylique et 140 cm³ d'eau, on décante la phase éthérée, puis on la lave par successivement 140 cm³ d'eau, 140 cm³ d'une solution aqueuse d'hydronénosulfate de sodium 1M., 140 cm³ dune solution saturée de chlorure de sodium. La phase organique est séchée sur sulfate de sodium, évaporée sous pression réduite (5 kPa) à une température voisine de 40°C. On obtient 7,88 g de N-Boc-(4-méthoxyphényl)glycinate de méthyle, sous forme d'une huile qui cristallise rapidement (R^{f}=0,28 dans le mélange cyclohexane-éther diéthylique 30/20 en volumes, sur plaque de silice Merck 60F_{254^{R}}).

N-Boc-(4-hydroxyphényl)glycine : A une solution agitée de 7,55 g de 4hydroxyphénylglycine dans 53,2 cm³ d'une solution aqueuse de soude 1N, on ajoute à une température voisine de 20°C 12 g de di-*tert*-butyle dicarbonate, puis le mélange est agité pendant 4 heures à une température voisine de 15°C. Après abandon pendant 16 heures du milieu réactionnel à une température voisine de 20°C, celui-ci est lavé par 2 fois 30 cm³ d'éther diéthylique. La phase aqueuse est décantée puis additionnée, sous agitation, de 73 cm³ d'une solution aqueuse d'hydronénosulfate de sodium 1M. Le mélange est extrait par 2 fois 135 cm³ de dichlorométhane. Les extraits organiques sont réunis, séchés sur sulfate de sodium, concentrés sous pression réduite (5 kPa) à une température voisine de 40°C. On obtient une meringue blanche que l'on reprend par 60 cm³ d'eau, et que l'on essore. Le solide obtenu est séché sous pression réduite (10 Pa) à une température voisine de 20°C. On obtient 9,26 g de N-Boc-(4-hydroxyphényl)glycine, sous forme d'un solide blanc fondant à 110°C.

### Exemple 3

### Chlorhydrate de (-)-(4R)-4-(4-méthoxyphényl)-4,5-dihydro-1,3-thiazol-2-ylamine

On procède dans les conditions de l'exemple 1 à partir de 2,5 g de N-*(tert*-butyl)-N'-[(1R)-1-(4-méthoxyphényl)-2-hydroxyéthyl]thiourée, dans 25 cm³ d'acide chlorhydrique aqueux 6N pendant 3 heures à une température voisine de 100°C. La masse réactionnelle est évaporée sous pression réduite (5 kPa) à une température voisine de 50°C. Au résidu obtenu, on ajoute 15 cm³ de soude aqueuse 1N et 5 cm³ d'eau. Le mélange est extrait par 20 cm³ d'acétate d'éthyle, puis la phase organique est concentrée comme ci-dessus. Le produit obtenu est purifié par chromatographie, sous une pression d'argon de 50 kPa, sur une colonne de gel de silice (granulométrie 40-63 µ ; masse silice 80 g), en éluant par un mélange d'acétate d'éthyle-méthanol (80/20 en volumes), et en recueillant des fractions d'environ 10 cm³. Les fractions 10 à 16 sont réunies, puis évaporées sous pression réduites (5 kPa) à une température voisine de 40°C. On obtient 0,23 g d'une laque dont on fait le chlorhydrate de la façon suivante : le produit est solubilisé dans 2,5 cm³ d'acétone et 1 cm³ d'éther diéthylique, puis on y ajoute 2 cm³ d'éther chlorhydrique 4,5 N. Un produit cristallisé précipite. Il est filtré, lavé 3 fois à l'acétone, puis séché à une température voisine de 40°C sous pression réduite (10 Pa). On obtient 0,09 g de chlorhydrate de (-)-(4R)-4-(4-méthoxyphényl)-4,5-dihydro-1,3-thiazol-2-ylamine, sous forme d'un solide blanc fondant à 236°C. (α_{D}²⁰ = -79,6 +/-1,3 dans le méthanol à 0,5%).

N-(*tert*-Butyl)-N'-[(1R)-1-(4-méthoxyphényl)-2-hydroxyéthyl]thiourée : On procède dans les conditions de l'exemple 1 à partir de 5,78 g de (2R)-2-amino-2-(4-méthoxyphényl)-1-éthanol et 6,48 cm³ d'isothiocyanate de *tert*-butyle dans 73 cm³ d'éthanol pendant 17h30 à une température voisine de 20°C. La masse réactionnelle est évaporée sous pression réduite (5 kPa) à une température voisine de 40°C, puis le résidu solide obtenu est broyé dans 45 cm³ d'eau, filtré, lavé par 2 fois 20 cm³ d'eau et 2 fois 25 cm³ d'éther diéthylique. Les cristaux sont séchés à l'étuve sous pression réduite (10 Pa) à une température voisine de 45°C. On obtient 4,9 g de N-*(tert*-butyl)-N'-[(1R)-1-(4-méthoxyphényl)-2-hydroxyéthyl]thiourée, sous forme d'un solide blanc (R_{f}=0,60 dans l'acétate d'éthyle, sur plaque de silice Merck 60F_{254^{R}}).

(2R)-2-Amino-2-(4-méthoxyphényl)-1-éthanol : On procède dans les conditions de l'exemple 2 à partir de 12,45 g de (1R)-1-(4-méthoxyphényl)-2-hydroxyéthylcarbamate de tert-butyle, dans 130 cm³ de méthanol chlorhydrique 2,5N, sous agitation pendant 1h30, à une température voisine de 20°C, puis 30 minutes à une température voisine de 30°C. La masse réactionnelle est évaporée sous pression réduite (5 kPa) à une température voisine de 40°C. Au résidu obtenu on ajoute 61 cm³ d'une solution aqueuse d'hydrogénocarbonate de sodium à 5%, puis le mélange est extrait par 3 fois 100 cm³ de dichlorométhane. La phase aqueuse est décantée, puis évaporée comme ci-dessus. Le solide obtenu est repris par 65 cm³ d'une solution aqueuse de soude 1N, et la solution résultante est ramenée aux 2/3 de son volume par concentration dans les conditions de ci-dessus. Le mélange est extrait par 3 fois 100 cm³ de dichlorométhane. Les extraits sont réunis, séchés sur sulfate de magnésium, filtrés, évaporés sous pression réduite (5 kPa) à une température voisine de 40°C. Les cristaux obtenus sont séchés sous pression réduite (5 kPa) aux environs de 20°C. On obtient 5,86 g de (2R)-2-amino-2-(4-méthoxyphényl)-1-éthanol, sous forme d'un solide blanc fondant à 103°C.

(1R)-1-(4-Méthoxyphényl)-2-hydroxyéthylcarbamate de tert-butyle : On procède dans les conditions de l'exemple 2 à partir de 15,6 g de N-Boc-D-(4-méthoxyphényl)glycinate de méthyle solubilisés dans 70 cm³ de tétrahydrofuranne anhydre, et en présence de 4,48 g de chlorure de lithium. L'addition par fractions de 3,98 g de borohydrure de sodium s'effectue à une température voisine de 0°C. Après addition de 148 cm³ d'éthanol absolu à cette même température, on laisse remonter la température au voisinage de 20°C, et l'agitation est poursuivie pendant 20 heures. Après refroidissement de la masse réactionnelle à une température voisine de 5°C, on ajoute 98 cm³ d'une solution aqueuse d'hydrogénosulfate de sodium 1M. Le mélange est agité pendant 3 heures, puis on l'évapore sous pression réduite (5 kPa) à une température voisine de 40°C. Le résidu est repris par 220 cm³ de dichlorométhane, 70 cm³ d'une solution aqueuse d'hydrogénosulfate de sodium 1M et 30 cm³ d'eau. Le mélange est agité puis décanté. La phase organique est séparée tandis que la phase aqueuse est extraite par 2 fois 60 cm³ de dichlorométhane. Les extraits organiques sont réunis puis séchés sur sulfate de sodium, filtrés, évaporés sous pression réduite (5 kPa) à une température voisine de 40°C. On obtient un solide que l'on reprend par 50 cm³ de cyclohexane ; puis les cristaux sont essorés, séchés à l'étuve sous pression réduite (10 Pa) à une température voisine de 60°C. On obtient 12,47 g de (1R)-1-(4-méthoxyphényl)-2-hydroxyéthylcarbamate de tert-butyle, sous forme d'un solide blanc fondant à 138°C.

N-Boc-D-(4-méthoxyphényl)glycinate de méthyle : On procède comme dans l'exemple 2 à partir d'une solution de 30,8 g de N-Boc-D-(4-hydroxyphényl)glycine dans 418 cm³ de diméthylformamide anhydre, refroidie à une température voisine de 0°C, à laquelle on ajoute 17,5 cm³ d'iodure de méthyle en présence de 39 g de carbonate de potassium. Le milieu réactionnel est ramené à une température voisine de 20°C, puis agité pendant 22 heures à cette même température. On ajoute au mélange 1 litre d'éther diéthylique et 500 cm³ d'eau. La phase éthérée est séparée par décantation, lavée par 500 cm³ d'eau, puis évaporée sous pression réduite (5 kPa) à une température voisine de 40°C. On obtient 31,53 g de N-Boc-D-(4-méthoxyphényl)glycinate de méthyle, sous forme d'une huile jaune qui cristallise rapidement (R^{f}=0,30 dans le cyclohexane-éther diéthylique, 30/20, sur plaque de silice Merck 60F_{254^{R}}).

N-Boc-D-(4-hydroxyphényl)glycine : On opère dans les conditions de l'exemple 2 à partir d'une solution de 30,2 g de D-(-)-(4-hydroxyphényl)glycine dans 213 cm³ d'une solution aqueuse de soude 1N, 48 g de di-*tert*-butyle dicarbonate, agités pendant 4 heures à une température voisine de 20°C. Le milieu réactionnel est lavé par 2 fois 120 cm³ d'éther diéthylique, puis la phase aqueuse est décantée, additionnée de 392 cm³ d'une solution aqueuse d'hydrogénosulfate de sodium 1M, quantité nécessaire pour obtenir un pH voisin de 1. On ajoute 450 cm³ de dichlorométhane, puis après agitation du mélange on sépare par décantation la phase organique. La phase aqueuse est extraite encore 2 fois par 450 cm³ de dichlorométhane. Les extraits organiques sont réunis, séchés sur sulfate de sodium puis concentrés sous pression réduite (5 kPa) à une température voisine de 40°C. On obtient un résidu que l'on reprend par 240 cm³ d'eau. Les cristaux qui en résultent sont essorés, puis séchés sous pression réduite (10 Pa) à une température voisine de 40°C. On obtient 9,59 g de N-Boc-D-(4-hydroxyphényl)glycine, sous forme d'un solide blanc [Spectre de R.M.N.¹H (300 MHz, (CD₃)₂SO d6, δ en ppm) : 1,38 (s : 9H) ; 4,98 (d, J = 9 Hz : 1H) ; 6,73 (d, J = 8 Hz : 2H) ; 7,20 (d, J = 8 Hz : 2H) ; 7,41(d, J = 9Hz, 1H) ; 9,48 (mf : 1H)].

### Exemple 4

### Chlorhydrate de (-)-(4R)-4-phényl-4,5-dihydro-1,3-thiazol-2-ylamine

On procède dans les conditions de l'exemple 1 à partir de 2,4 g de N-(*tert*-butyl)-N'-[(1R)-2-hydroxy-1-phényléthyl]thiourée dans 24 cm³ d'acide chlorhydrique aqueux 6N pendant 1heure 30 minutes à une température voisine de 100°C. Après refroidissement du milieu à une température voisine de 0°C, le précipité formé est filtré, puis le gâteau est lavé par 7 cm³ d'acide chlorhydrique aqueux 6N et 2 fois 15 cm³ d'éther diéthylique. Le solide obtenu est séché à l'étuve sous pression réduite (10 Pa) à une température voisine de 60°C. On obtient 1,6 g de chlorhydrate de (-)-(4R)-4-phényl-4,5-dihydro-1,3-thiazol-2-ylamine, sous forme d'un solide blanc fondant à 260°C (α_{D}²⁰ = -109,6 +/- 1,3 dans le méthanol à 1%).

N-(*tert*-Butyl)-N'-[(1R)-2-hydroxy-1-phényléthyl]thiourée : On procède dans les conditions de l'exemple 1 à partir de 2,74 g de (R)-(-)-2-amino-2-phényléthanol, 2,8 cm³ d'isothiocyanate de *tert*-butyle dans 15 cm³ d'éthanol pendant 20 heures à une température voisine de 20°C. Le milieu réactionnel est concentré sous pression réduite (5 kPa) à une température voisine de 50°C. On obtient une huile jaune que l'on solubilise dans 100 cm³ d'acétate d'éthyle, puis la solution organique est lavée par 2 fois 50 cm³ d'une solution aqueuse d'hydrogénocarbonate de sodium et 2 fois par une solution aqueuse de chlorure de sodium. Après séchage sur sulfate de sodium, la solution organique est filtrée puis concentrée sous pression réduite (5 kPa) à une température voisine de 40°C. On obtient 4,9 g de N-(*tert*-butyl)-N'-[(1R)-2-hydroxy-1-phényléthyl]thiourée, sous forme d'un solide jaune fondant à 98°C.

### Exemple 5

### Chlorhydrate de (4RS)-4-benzylsulfanylméthyl-4,5-dihydro-thiazol-2-ylamine

Une suspension de 0,1 g de N-*tert*-butyl-N'-[(4RS)-4-benzylsulfanylméthyl-4,5-dihydro-thiazol-2-yl]-amine dans 5 cm³ d'acide chlorhydrique 5N est chauffée à une température voisine de 100°C pendant 2 heures. Après refroidissement à température ambiante le mélange réactionnel est concentré sous pression réduite (1 kPa) à une température voisine de 50°C. L'huile obtenue est triturée dans 5 cm³ d'éther diéthylique, puis le solide obtenu est filtré et séché au dessiccateur sous pression réduite (0,1 kPa) à une température voisine de 40°C. On obtient 0,06 g de chlorhydrate de (4RS)-4-benzylsulfanyhnéthyl-4,5-dihydro-thiazol-2-ylamine sous forme de solide crème [Spectre de R.M.N. ¹H (300 MHz, (CD₃)₂SO d6, δ en ppm) : 2,74 (d, J = 6 Hz : 2H) ; 3,37 (dd, J =11,5 et 5,5 Hz : 1H) ; 3,71 (dd, J = 11,5 et 8 Hz : 1H) ; 3,85 (s : 2H) ; 4,45 (mt : 1H) ; de 7,25 à 7,45 (mt : 5H) ; de 9,00 à 9,90 (mf très étalé : 2H) ; 10,03 (mf : 1H)].

N-*tert*-Butyl-N'-[(4RS)-4-benzylsulfanylméthyl-4,5-dihydro-thiazol-2-yl]-amine : A une solution de 0,39 g de N-[(4RS)-4-p-toluènesulfonylméthyl-4,5-dihydro-thiazol-2-yl]-carbamate de *tert*-butyle dans 20 cm³ d'acétonitrile, on ajoute sous agitation, à une température voisine de 20°C, 0,28 g de carbonate de potassium et 0,13 cm³ de benzyl mercaptan. Après 40 heures d'agitation à une température voisine de 20°C le mélange réactionnel est filtré. Le filtrat est concentré sous pression réduite (1 kPa) à une température voisine de 50°C. Le résidu obtenu est purifié par chromatographie sous pression atmosphérique sur une colonne de gel de silice (granulométrie 40-60µ), en éluant par un mélange de cyclohexane-acétate d'éthyle (75/25 en volumes) et en recueillant des fractions de 20 cm³. Les fractions contenant le produit attendu sont réunies puis concentrées dans les conditions de ci-dessus. On obtient 11 g de N*-tert*-butyl-N'-[(4RS)-4-benzylsulfanylméthyl-4,5-dihydro-thiazol-2-yl]-amine sous forme d'une laque crème [Spectre de R.M.N. ¹H (300 MHz, (CD₃)₂SO d6, δ en ppm) : 1,41 (s : 9H) ; de 2,50 à 2,70 (mt : 2H); 3,01 (dd, J = 11 et 6 Hz : 1H) ; de 3,25 à 3,40 (mt : 1H) ; 3,82 (s : 2H) ; 4,13 (mt : 1H) ; de 7,20 à 7,40 (mt : 5H) ; de 9,40 à 9,80 (mf très étalé : 1H)].

N-[(4RS)-4-p-Toluènesulfonylméthyl-4,5-dihydro-thiazol-2-yl]-carbamate de *tert* butyle : Une solution de 0,8 g de N-[(4RS)-4-hydroxyméthyl-4,5-dihydro-thiazol-2-yl]-carbamate de *tert*-butyle, de 0,76 g de chlorure de p-toluènesulfonyle et de 0,56 cm³ de triéthylamine dans 25 cm³ de dichlorométhane est agitée pendant 16 heures à une température voisine de 20°C. La solution obtenue est concentrée sous pression réduite (1 kPa) à une température voisine de 40°C. Le résidu d'évaporation obtenu est purifié par chromatographie sous pression atmosphérique sur une colonne de gel de silice (granulométrie 60-200µ ; diamètre 2 cm ; hauteur 25 cm), en éluant par un mélange de cyclohexane-acétate d'éthyle (70/30 en volumes) et en recueillant des fractions de 30 cm³. Les fractions contenant le produit attendu sont réunies puis concentrées dans les conditions de ci-dessus. On obtient 0,8 g de N-[(4RS)-4-p-toluènesulfonylinéthyl-4,5-dihydro-thiazol-2-yl]-carbamate de *tert*-butyle sous forme d'un solide blanc [Spectre de R.M.N. ¹H (300 MHz, CDCl₃, δ en ppm) : 1,48 (s : 9H) ; 2,46 (s : 3H) ; 3,10 (dd, J = 11,5 et 5,5 Hz : 1H) ; 3,33 (dd, J = 11,5 et 8,5 Hz : 1H) ; 3,97 (dd, J = 9,5 et 8 Hz : 1H) ; 4,06 (dd, J = 9,5 et 4,5 Hz : 1H) ; 4,43 (mt : 1H) ; 7,36 (d, J = 8 Hz : 2H) ; 7,80 (d, J = 8 Hz : 2H) ; de 8,50 à 9,40 (mf très étalé : 1H)].

N-[(4RS)-4-Hydroxyméthyl-4,5-dihydro-thiazol-2-yl]-carbamate de *tert*-butyle : A une solution de 2g de 2-[(*tert*-butoxycarbonyl)imino]-(4RS)-4-[(*tert*-butoxycarbonyl)oxy]-méthyl-1,3-thiazolidine-3-carboxylate de *tert*-butyle dans 20 cm³ de méthanol on ajoute 10 cm³ de soude aqueuse N puis on agite à une température voisine de 20°C pendant 4 heures. Le mélange réactionnel est concentré sous pression réduite (1 kPa) à une température voisine de 50°C. Le résidu obtenu est repris par 30 cm³ eau, filtré, lavé par de l'acétate d'éthyle puis de l'eau. On obtient 0,37 g de N[4-(R,S)-(hydroxy-méthyl)-4,5-dihydro-thiazol-2-yl] carbamate de *tert*-butyle sous forme de solide blanc [Spectre de masse : DCI m/z=233, MH⁺ m/z=177 (M-C₄H₇)⁺].

2-[(*tert*-Butoxycarbonyl)imino]-(4RS)-4-[(*tert*-butoxycarbonyl)oxy]-méthyl-1,3-thiazolidine-3-carboxylate de *tert*-butyle : A une solution de 1,98 g de (4RS)-4-hydroxyméthyl-4,5-dihydro-thiazol-2-ylamine dans 20 cm³ de dichlorométhane on ajoute 10,91 g de di-*tert*-butyldicarbonate et 2,81 cm³ de triéthylamine puis on agite à une température voisine de 20°C. Au bout de 4 heures, on ajoute à nouveau 3 cm³ de triéthylamine, puis on agite pendant 16 heures à une température voisine de 20°C. On ajoute 50 cm³ d'eau au mélange réactionnel, on décante, sèche sur sulfate de magnésium, filtre et concentre sous pression réduite (1 kPa), à une température voisine de 40°C. On obtient 7 g de 2-[(*tert*-butoxycarbonyl)imino]-(4RS)-4-[(*tert*-butoxycarbonyl)oxy]-méthyl-1,3-thiazolidine-3-carboxylate de *tert*-butyle sous forme d'une huile [Spectre de masse : DCI m/z=433, MH⁺ m/z=333 (M-C₅H₇O₂)⁺].

(4RS)-4-Hydroxyméthyl-4,5-dihydro-thiazol-2-ylamine : Une solution de 90 g de 1-*tert*-butyl-3-(2-hydroxy-1-hydroxyméthyl-éthyl)-thiourée dans 500 cm³ d'acide chlorhydrique 6N est agitée à une température voisine de 100°C. Après 3 heures, le mélange réactionnel est refroidi au voisinage de 20°C puis est concentré sous pression réduite (1 kPa) à une température voisine de 50°C. Le résidu obtenu est repris par 100 cm³ d'eau, alcalinisé par 100 cm³ de soude 5N puis concentré comme précédemment. L'huile obtenue est agitée pendant 20 heures à une température voisine de 20°C dans 300 cm³ d'éthanol, filtrée, lavée par 5 fois 50 cm³ d'éthanol puis par 3 fois 100 cm³ de méthanol. Les différents filtrats sont réunis, évaporés sous pression réduite (1 kPa), à une température voisine de 40°C puis cristallisés dans 400 cm³ d'éthanol pour obtenir 31 g de (4RS)-4-hydroxyméthyl-4,5-dihydro-thiazol-2-ylamine sous forme d'un solide blanc fondant à 122°C [Spectre infra-rouge (KBr): 3311; 3164; 1648; 1601; 1349; 1051 et 982 cm⁻¹].

1-*tert*-Butyl-3-(2-hydroxy-1-hydroxyméthyl-éthyl)-thiourée : A une solution de 14,6 g de 2 amino-1,3-propane-diol dans 245 cm³ d'éthanol on ajoute 30,4 cm³ d'isothiocyanate de butyle puis on agite à une température voisine de 20°C pendant 94 heures. Le milieu réactionnel est concentré sous pression réduite (5 kPa), à une température voisine de 40°C, trituré dans un mélange de 160 cm³ d'éther de pétrole et de 26 cm³ d'éthanol, filtré puis séché au dessiccateur sous pression réduite (0,1 kPa) à une température voisine de 60°C. On obtient 30 g de 1-*tert*-butyl-3-(2-hydroxy-1-hydroxyméthyl-éthyl)-thiourée sous forme de solide blanc [Spectre de R.M.N. ¹H (250 MHz, (CD₃)₂SO d6, δ en ppm) : 1,42 (s : 9H) ; 3,38 (mt : 2H) ; 3,54 (mt : 2H) ; 4,17 (mf : 1H) ; 4,70 (t, J = 5 Hz : 2H) ; 7,08 (d, J = 8 Hz : 1H) ; 7,38 (s : 1H)].

Les médicaments selon l'invention sont constitués par un composé de formule (I) ou un isomère ou un tautomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention de la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis, la croissance de certaines formes de tumeurs comme par exemple les épithéliomes, les adénocarcinomes ou les sarcomes, et les infections par des bactéries intracellulaires ou extracellulaires, gram-plus ou gram-moins.

Les doses dépendent de l'effet recherché, de la durée du traitement et de la voie d'administration utilisée; elles sont généralement comprises entre 1 mg et 100 mg par jour par voie orale pour un adulte avec des doses unitaires allant de 0,5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

La présente invention concerne également l'utilisation d'un composé de formule (I) ses racémique, énantiomères, diastéréoisomères, leurs mélanges, sa forme tautomère et ses sels pharmaceutiquement acceptables pour la préparation de compositions pharmaceutiques destinées à prévenir et traiter les maladies dans lesquelles une production anormale de monoxyde d'azote (NO) par induction de NO-synthase inductible (NOS-2) est impliquée.

La présente invention concerne également la méthode de prévention et de traitement des maladies dans lesquelles une production anormale de monoxyde d'azote (NO) par induction de NO-synthase inductible (NOS-2 ou iNOS) est impliquée par administration d'un composé de formule (I) ses racémique, énantiomères, diastéréoisomères et leurs mélanges, son tautomère et ses sels pharmaceutiquement acceptables.

## Revendications

1. Composés de formule : 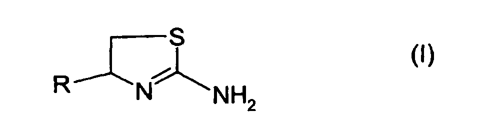 dans laquelle R représente un radical -alk-S-alk-Ar, phényle ou phényle substitué par alcoxy (1-6C en chaîne droite ou ramifiée) ou halogène, Ar représente un radical phényle et alk un radical alkylène (1-6C en chaîne droite ou ramifiée), ses racémique, énantiomères, diastéréoisomères et leurs mélanges, son tautomère et ses sels pharmaceutiquement acceptables pour son utilisation comme médicament.

2. Composés selon la revendication 1 de formule (I) dans laquelle R est un radical phényle ou phényle monosubstitué par alcoxy (1-6C en chaîne droite ou ramifiée) ou un atome d'halogène, ses racémique, énantiomères, diastéréoisomères et leurs mélanges, son tautomère et ses sels pharmaceutiquement acceptables pour son utilisation comme médicament.

3. Composés selon la revendication 2 de formule (I) dans laquelle R est un phényle monosubstitué par alcoxy (1-6C en chaîne droite ou ramifiée) ou un atome d'halogène en position -3 ou -4, ses racémique, énantiomères, diastéréoisomères et leurs mélanges, son tautomère et ses sels pharmaceutiquement acceptables pour son utilisation comme médicament.

4. Composés selon l'une des revendications 1 à 3 de formule (I) dans laquelle l'atome d'halogène est un atome de brome pour son utilisation comme médicament.

5. Composés selon l'une des revendications 1 à 3 de formule (I) dans laquelle l'alcoxy est un radical méthoxy pour son utilisation comme médicament.

6. Composés selon l'une des revendications 1 à 5 de formule (I) choisi parmi les composés suivants :
4-(3-bromophényl)-4,5-dihydro-1,3-thiazol-2-ylamine
4-(4-méthoxyphényl)-4,5-dihydro-1,3-thiazol-2-ylamine
4-phényl-4,5-dihydro-1,3-thiazol-2-ylamine
4-(benzylsulfanylméthyl)-4,5-dihydro-1,3-thiazol-2-ylamine
ses racémique, énantiomères, diastéréoisomères et leurs mélanges, son tautomère et ses sels pharmaceutiquement acceptables pour son utilisation comme médicament.

7. Composés selon l'une des revendications 1 à 5 de formule (I) choisi parmi les composés suivants :
(4RS)-4-(3-bromophényl)-4,5-dihydro-1,3-thiazol-2-ylamine
4-(4-méthoxyphényl)-4,5-dihydro-1,3-thiazol-2-ylamine
(-)-(4R) -4-(4-méthoxyphényl)-4,5-dihydro-1,3-thiazol-2-ylamine
(-)-(4R) -4-phényl-4,5-dihydro-1,3-thiazol-2-ylamine
son tautomère et ses sels pharmaceutiquement acceptables pour son utilisation comme médicament.

8. Composés de formule : 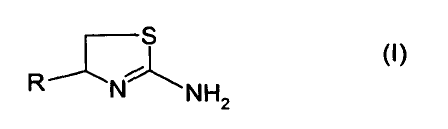 dans laquelle R représente un radical -alk-S-alk-Ar, phényle substitué par alcoxy (1-6C en chaîne droite ou ramifiée) ou halogène, Ar représente un radical phényle et alk un radical alkylène (1-6C en chaîne droite ou ramifiée), leurs racémiques, énantiomères, diastéréoisomères et leurs mélanges, leurs tautomères et leurs sels pharmaceutiquement acceptables.

9. Composés selon la revendication 8 choisis parmi les suivants :
4-(3-bromophényl)-4,5-dihydro-1,3-thiazol-2-ylamine
4-(4-méthoxyphényl)-4,5-dihydro-1,3-thiazol-2-ylamine
4-(benzylsulfanylméthyl)-4,5-dihydro-1,3-thiazol-2-ylamine
leurs racémiques, énantiomères, diastéréoisomères et leurs mélanges, leurs tautomères et leurs sels pharmaceutiquement acceptables.

10. Composés de formule (I) selon la revendication 8 choisis parmi les suivants :
(4RS)-4-(3-bromophényl)-4,5-dihydro-1,3-thiazol-2-ylamine
4-(4-méthoxyphényl)-4,5-dihydro-1,3-thiazol-2-ylamine
(-)-(4R) -4-(4-méthoxyphényl)-4,5-dihydro-1,3-thiazol-2-ylamine
leurs tautomères et leurs sels pharmaceutiquement acceptables.

11. Procédé de préparation des composés de formule (I) selon la revendication 8 **caractérisé en ce que** l'on cyclise un dérivé de formule : 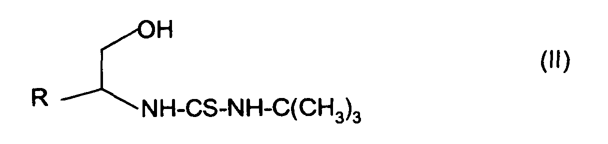 dans laquelle R a les mêmes significations que dans la revendication 8, isole le produit et le transforme éventuellement en sel pharmaceutiquement acceptable.

12. Procédé de préparation des composés de formule (I) selon la revendication 8 pour lesquels R est un radical -alk(1C)-S-alk-Ar **caractérisé en ce que** l'on fait réagir un dérivé de formule : 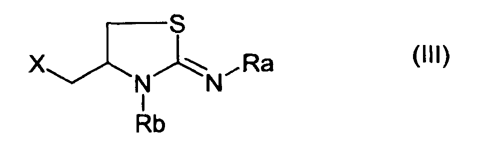 dans laquelle X est un atome d'halogène ou un radical tosyle, Ra et Rb sont des atomes d'hydrogène ou des groupes protecteurs de la fonction amine, avec un dérivé de formule HS-alk-Ar dans laquelle Ar représente un radical phényle et alk représente un radical alkylène (1-6C en chaîne droite ou ramifiée), et si nécessaire déprotège la fonction amine, isole le produit et le transforme éventuellement en sel pharmaceutiquement acceptable.

13. Utilisation d'un composé de formule : 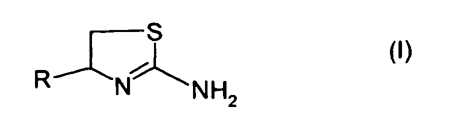 dans laquelle R représente un radical -alk-S-alk-Ar, phényle ou phényle substitué par alcoxy (1-6C en chaîne droite ou ramifiée) ou halogène, Ar représente un radical phényle et alk un radical alkylène (1-6C en chaîne droite ou ramifiée), ses racémique, énantiomères, diastéréoisomères et leurs mélanges, son tautomère et ses sels pharmaceutiquement acceptables pour la préparation d'un médicament destiné à prévenir et traiter les maladies dans lesquelles une production anormale de monoxyde d'azote (NO) par induction de NO-synthase inductible (NOS-2) est impliquée.

## Patentansprüche

1. Verbindungen der Formel (I) 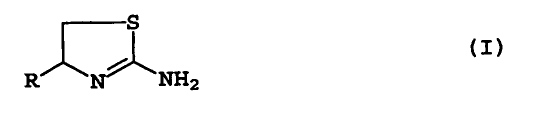 in der
R einen Rest -alk-S-alk-Ar, Phenyl oder Phenyl darstellt, substituiert durch Alkoxy (1 bis 6 Kohlenstoffatome in gerader oder verzweigter Kette) oder Halogen, Ar ein Rest Phenyl ist und alk einen Rest Alkylen (1 bis 6 Kohlenstoffatome in gerader oder verzweigter Kette) bedeutet, ihre Racemate, Enantiomeren, Diastereoisomeren und ihre Mischungen, ihre Tautomeren und ihre pharmazeutisch akzeptablen Salze für ihre Verwendung als Arzneimittel.

2. Verbindungen nach Anspruch 1 der Formel (I), in der R ein Rest Phenyl oder Phenyl ist, monosubstituiert durch Alkoxy (1 bis 6 Kohlenstoffatome in gerader oder verzweigter Kette) oder ein Halogenatom, ihre Racemate, Enantiomeren, Diastereoisomeren und ihre Mischungen, ihre Tautomeren und ihre pharmazeutisch akzeptablen Salze für ihre Verwendung als Arzneimittel.

3. Verbindungen nach Anspruch 2 der Formel (I), in der R ein Phenyl ist, monosubstituiert durch Alkoxy (1 bis 6 Kohlenstoffatome in gerader oder verzweigter Kette) oder ein Halogenatom in Position -3 oder -4, ihre Racemate, Enantiomeren, Diastereoisomeren und ihre Mischungen, ihre Tautomeren und ihre pharmazeutisch akzeptablen Salze für ihre Verwendung als Arzneimittel.

4. Verbindungen nach einem der Ansprüche 1 bis 3 der Formel (I), in der das Halogenatom ein Bromatom ist, für ihre Verwendung als Arzneimittel.

5. Verbindungen nach einem der Ansprüche 1 bis 3 der Formel (I), in der das Alkoxy ein Rest Methoxy ist, für ihre Verwendung als Arzneimittel.

6. Verbindungen nach einem der Ansprüche 1 bis 5 der Formel (I), ausgewählt unter den folgenden Verbindungen:
4-(3-Bromphenyl)-4,5-dihydro-1,3-thiazol-2-yl-amin
4-(4-Methoxyphenyl)-4,5-dihydro-1,3-thiazol-2-yl-amin
4-Phenyl-4,5-dihydro-1,3-thiazol-2-yl-amin
4-(Benzylsulfanylmethyl)-4,5-dihydro-1,3-thiazol-2-yl-amin,
ihre Racemate, Enantiomeren, Diastereoisomeren und ihre Mischungen, ihre Tautomeren und ihre pharmazeutisch akzeptablen Salze für ihre Verwendung als Arzneimittel.

7. Verbindungen nach einem der Ansprüche 1 bis 5 der Formel (I), ausgewählt unter den folgenden Verbindungen:
(4RS)-4-(3-Bromphenyl)-4,5-dihydro-1,3-thiazol-2-yl-amin
4-(4-Methoxyphenyl)-4,5-dihydro-1,3-thiazol-2-yl-amin
(-)-(4R)-4-(4-Methoxyphenyl)-4,5-dihydro-1,3-thiazol-2-yl-amin
(-)-(4R)-4-Phenyl-4,5-dihydro-1,3-thiazol-2-yl-amin,
ihre Tautomeren und ihre pharmazeutisch akzeptablen Salze für ihre Verwendung als Arzneimittel.

8. Verbindungen der Formel 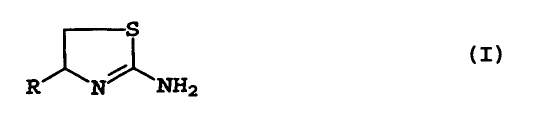 in der
R einen Rest -alk-S-alk-Ar oder Phenyl darstellt, substituiert durch Alkoxy (1 bis 6 Kohlenstoffatome in gerader oder verzweigter Kette) oder Halogen, Ar ein Rest Phenyl ist und alk einen Rest Alkylen (1 bis 6 Kohlenstoffatome in gerader oder verzweigter Kette) bedeutet, ihre Racemate, Enantiomeren, Diastereoisomeren und ihre Mischungen, ihre Tautomeren und ihre pharmazeutisch akzeptablen Salze.

9. Verbindungen nach Anspruch 8, ausgewählt unter den folgenden:
4-(3-Bromphenyl)-4,5-dihydro-1,3-thiazol-2-yl-amin
4-(4-Methoxyphenyl)-4,5-dihydro-1,3-thiazol-2-yl-amin
4-(Benzylsulfanylmethyl)-4,5-dihydro-1,3-thiazol-2-yl-amin,
ihre Racemate, Enantiomeren, Diastereoisomeren und ihre Mischungen, ihre Tautomeren und ihre pharmazeutisch akzeptablen Salze.

10. Verbindungen der Formel (I) nach Anspruch 8, ausgewählt unter den folgenden:
(4RS)-4-(3-Bromphenyl)-4,5-dihydro-1,3-thiazol-2-yl-amin
4-(4-Methoxyphenyl)-4,5-dihydro-1,3-thiazol-2-yl-amin
(-)-(4R)-4-(4-Methoxyphenyl)-4,5-dihydro-1,3-thiazol-2-yl-amin,
ihre Tautomeren und ihre pharmazeutisch akzeptablen Salze.

11. Verfahren zur Herstellung der Verbindungen der Formel (I) nach Anspruch 8, **dadurch gekennzeichnet, daß** man ein Derivat der Formel (II) 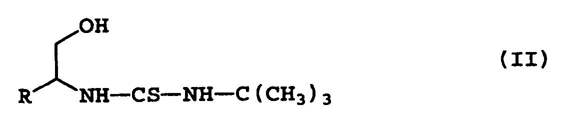 in der
R die gleiche Bedeutungen wie in Anspruch 8 besitzt, cyclisiert, das Produkt isoliert und gegebenenfalls in ein pharmazeutisch akzeptables Salz überführt.

12. Verfahren zur Herstellung der Verbindungen der Formel (I) nach Anspruch 8, worin R ein Rest -alk(1C)-S-alk-Ar ist, **dadurch gekennzeichnet, daß** man ein Derivat der Formel (III) 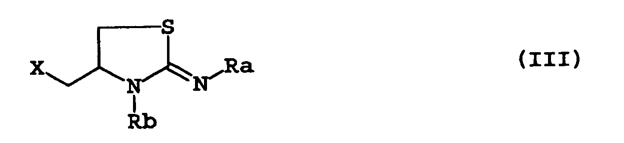 in der X ein Halogenatom oder ein Rest Tosyl ist, Ra und Rb Wasserstoffatome oder Schutzgruppen für die Aminfunktion darstellen, mit einem Derivat der Formel HS-alk-Ar, in der Ar ein Rest Phenyl ist und alk einen Rest Alkylen (1 bis 6 Kohlenstoffatome in gerader oder verzweigter Kette) darstellt, zur Reaktion bringt, und wenn erforderlich, die Schutzgruppen von der Aminfunktion entfernt, das Produkt isoliert und gegebenenfalls in ein pharmazeutisch akzeptables Salz überführt.

13. Verwendung einer Verbindung der Formel (I) 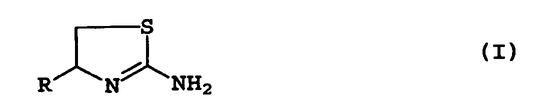 in der
R einen Rest -alk-S-alk-Ar, Phenyl oder Phenyl darstellt, substituiert durch Alkoxy (1 bis 6 Kohlenstoffatome in gerader oder verzweigter Kette) oder Halogen, Ar ein Rest Phenyl ist und alk einen Rest Alkylen (1 bis 6 Kohlenstoffatome in gerader oder verzweigter Kette) bedeutet, sowie von ihren Racematen, Enantiomeren, Diastereoisomeren und ihren Mischungen, ihren Tautomeren und ihren pharmazeutisch akzeptablen Salzen zur Herstellung eines Arzneimittels, das vorgesehen ist für die Vorbeugung und Behandlung von Erkrankungen, bei denen eine anormale Produktion von Stickstoffmonoxid (NO) durch Induktion von induktibler NO-Synthase (NOS-2) einbezogen ist.

## Claims

1. A compound of formula: 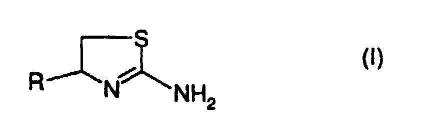 in which R represents an -alk-S-alk-Ar radical, a phenyl radical or a phenyl radical substituted with alkoxy (1-6C in a straight or branched chain) or halogen, Ar represents a phenyl radical and alk an alkylene radical (1-6C in a straight or branched chain), the racemic mixture, enantiomers and diastereoisomers thereof and mixtures thereof, the tautomer thereof and the pharmaceutically acceptable salts thereof for its use as medicament.

2. A compound of formula (I) as claimed in claim 1, in which R is a phenyl radical or a phenyl radical monosubstituted with alkoxy (1-6C in a straight or branched chain) or a halogen atom, the racemic mixture, enantiomers and diastereoisomers thereof and mixtures thereof, the tautomer thereof and the pharmaceutically acceptable salts thereof for its use as medicament.

3. A compound of formula (I) as claimed in claim 2, in which R is a phenyl monosubstituted with alkoxy (1-6C in a straight or branched chain) or a halogen atom in position 3 or 4, the racemic mixture, enantiomers and diastereoisomers thereof and mixtures thereof, the tautomer thereof and the pharmaceutically acceptable salts thereof for its use as medicament.

4. A compound of formula (I) as claimed in one of claims 1 to 3, in which the halogen atom is a bromine atom for its use as medicament.

5. A compound of formula (I) as claimed in one of claims 1 to 3, in which the alkoxy is a methoxy radical for its use as medicament.

6. A compound of formula (I) as claimed in one of claims 1 to 5, chosen from the following compounds:
4-(3-bromophenyl)-4,5-dihydro-1,3-thiazol-2-ylamine
4-(4-methoxyphenyl)-4,5-dihydro-1,3-thiazol-2-ylamine
4-phenyl-4,5-dihydro-1,3-thiazol-2-ylamine
4-(benzylsulfanylmethyl)-4,5-dihydro-1,3-thiazol-2-ylamine
the racemic mixture, enantiomers and diastereoisomers thereof and mixtures thereof, the tautomer thereof and the pharmaceutically acceptable salts thereof for its use as medicament.

7. A compound of formula (I) as claimed in one of claims 1 to 5, chosen from the following compounds:
(4RS)-4-(3-bromophenyl)-4,5-dihydro-1,3-thiazol-2-ylamine
4-(4-methoxyphenyl)-4,5-dihydro-1,3-thiazol-2-ylamine
(-)-(4R)-4-(4-methoxyphenyl)-4,5-dihydro-1,3-thiazol-2-ylamine
(-)-(4R)-4-phenyl-4,5-dihydro-1,3-thiazol-2-ylamine
the tautomer thereof and the pharmaceutically acceptable salts thereof for its use as medicament.

8. A compound of formula: 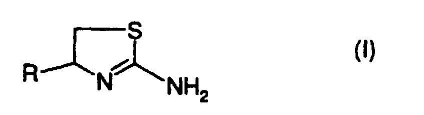 in which R represents an -alk-S-alk-Ar radical or a phenyl radical substituted with alkoxy (1-6 C in a straight or branched chain) or halogen, Ar represents a phenyl radical and alk an alkylene radical (1-6C in a straight or branched chain), the racemic mixtures, enantiomers and diastereoisomers thereof and mixtures thereof, the tautomers thereof and the pharmaceutically acceptable salts thereof.

9. A compound as claimed in claim 8, chosen from the following:
4-(3-bromophenyl)-4,5-dihydro-1,3-thiazol-2-ylamine
4-(4-methoxyphenyl)-4,5-dihydro-1,3-thiazol-2-ylamine
4-(benzylsulfanylmethyl)-4,5-dihydro-1,3-thiazol-2-ylamine
the racemic mixtures, enantiomers and diastereoisomers thereof and mixtures thereof, the tautomers thereof and the pharmaceutically acceptable salts thereof.

10. A compound of formula (I) as claimed in claim 8, chosen from the following:
(4RS)-4-(3-bromophenyl)-4,5-dihydro-1,3-thiazol-2-ylamine
4-(4-methoxyphenyl)-4,5-dihydro-1,3-thiazol-2-ylamine (-)-(4R)-4-(4-methoxyphenyl)-4,5-dihydro-1,3-thiazol-2-ylamine
(-)-(4R)-4-phenyl-4,5-dihydro-1,3-thiazol-2-ylamine
the tautomers thereof and the pharmaceutically acceptable salts thereof.

11. A process for preparing compounds of formula (I) as claimed in claim 8, wherein a derivative of formula: 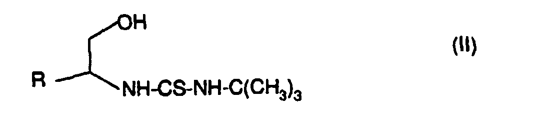 in which R has the same meanings as in claim 8, is cyclized, and the product is isolated and optionally converted into a pharmaceutically acceptable salt.

12. The process for preparing compounds of formula (I) as claimed in claim 8, for which R is a radical -alk(1C)-S-alk-Ar, wherein a derivative of formula: 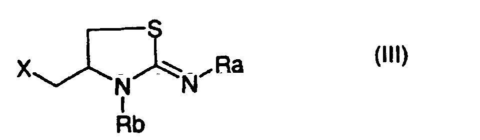 in which X is a halogen atom or a tosyl radical and Ra and Rb are hydrogen atoms or protecting groups for the amine function, is reacted with a derivative of formula HS-alk-Ar in which Ar represents a phenyl radical and alk represents an alkylene radical (1-6C in a straight or branched chain), and, if necessary, the amine function is deprotected and the product is isolated and optionally converted into a pharmaceutically acceptable salt.

13. Use of a compound of formula: 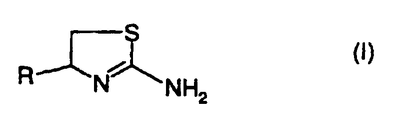 in which R represents an -alk-S-alk-Ar radical, a phenyl radical or a phenyl radical substituted with alkoxy (1-6C in a straight or branched chain) or halogen, Ar represents a phenyl radical and alk an alkylene radical (1-6C in a straight or branched chain), the racemic mixture, enantiomers and diastereoisomers thereof and mixtures thereof, the tautomer thereof and the pharmaceutically acceptable salts thereof for the preparation of a medicament intended for the prevention and treatment of illnesses which involve an abnormal production of nitrogen monoxide (NO) by inducing an inducible NO-synthase (NOS-2).
